# EUROPEAN PATENT SPECIFICATION

(11) **EP 0 353 516 B1**
(45) Date of publication and mention of the grant of the patent: **14.09.1994**
(21) Application number: 89112825.8
(22) Date of filing: 13.07.1989
(51) Int. Cl.: C12N 15/25, C12N 15/75, C12P 21/02, A61K 37/02

(54) **A method for the preparation of recombinant human beta interleukin-1 in the homogeneous form**
Methode zur Herstellung von rekombinantem menschlichem beta-Interleukin-1 in homogener Form
Méthode de préparation de la bêta-interleukine-1 humaine recombinante en forme homogène

(30) Priority: 05.08.1988 IT 2166788
(43) Date of publication of application: 07.02.1990
(73) Proprietor: ENIRICERCHE S.p.A., 20121 Milano (IT)
(72) Inventor: Velati Bellini, Ada, I-27100 Pavia (IT); Galli, Giuliano, I-00015 Monterotondo Roma (IT); Grandi, Guido, I-20090 Segrate S. Felice Milano (IT)
(74) Representative: Perani, Aurelio

(56) References cited:
- EP-A- 0 146 901
- EP-A- 0 281 530
- WO-A-88/01624
- GB-A- 2 186 580
- ABSTRACTS OF THE 88TH ANNUAL MEETING OF THE AMERICAN SOCIETY FOR MICROBIOLOGY, Miami Beach, Florida, 8th - 13th May 1988, page 151, abstract no. H-37; L.E.CHANG et al.: "Expression-secretion vectors for Bacillus"
- ANALYTICAL BIOCHEMISTRY, vol. 173, 1988, pages 440-444, Academic Press, Inc.; P.G. ZAWORSKI et al.: "Precipitation and recovery of proteins from culture supernatants using zinc"

## Description

The present invention relates to a method for the preparation of recombinant human beta interleukin-1 in the homogeneous form by the purification, in a single chromatographic step, of the soluble fraction obtained by the breaking of engineered Bacillus cells.

The invention also relates to a replicable recombinant DNA molecule with expression in Bacillus including the gene which codes for human beta interleukin-1 and a host micro-organism selected from the Bacillus genus, transformed by the recombinant DNA molecule.

Human beta interleukin-1, hereinafter referred to as beta hIL-1, is a protein with a molecular weight of approximately 17,000 daltons, belonging to the lymphomonokine family, a large group of proteins which modulate immune functions.

Beta hIL-1, which is largely produced by the cells of the monocyte-macrophage line, in fact seems to be one of the most important stimulators of the T lymphocytes (Oppenheim J. et al. Fed. Proc. 41, 257 (1982)), and contributes to the amplification of the immune response during the antigene-recognition stage by means of an effect on the T-helper and T-cytotoxic lymphocytes.

Moreover, beta hIL-1 performs many functions which are not actually immune functions but which can lead to a different protective mechanism which the host uses to overcome pathological conditions.

Finally, beta hIL-1 contributes to the activation of repair mechanisms in cases of tissue damage, stimulating the growth of fibroblasts.

Because of its multiple activity, the possibility of using hIL-1 in human therapy seems more and more attractive, particularly for the treatment of auto-immune diseases such as arthritis and lupus erythematosus and for healing wounds and burns.

This therefore requires the availability both of adequate quantities of human beta interleukin-1 and of a product with high purity and specific activity.

Methods known in the art for the preparation of hIL-1 comprise the cultivation under suitable conditions of human cells, for example such as peripheral blood leukocytes or monocytes or leukaemic cells obtained from patients affected by leukaemia and the purification of the product thus obtained by a combination of chromatographic techniques; in this connection, see Tagawa et al. (J. Immunol., 122, 2112-2118 (1979)); Lachman (Federation Proceedings, 42, 2639-2645 (1983)); Blyden et al. (J. Immunol., 118 1631-1638 (1977)).

However, the known methods are not completely satisfactory, particularly in view of the low production yields and the difficulty of purifying the protein thus produced to homogeneity.

The genes which code for alpha and beta human interleukin-1 respectively have recently been cloned and sequenced (March et al., Natura 315, 641-647 (1985); EP 200986, published on 12th November 1986) and this has enabled the development of methods for the preparation of the proteins with high yields.

More particularly, according to the known methods, human beta interleukin-1 is produced by the culture of strains of Escherichia coli (E. coli) transformed by a recombinant DNA molecule containing the gene which codes for the protein and the subsequent separation and purification of the recombinant product thus expressed.

However, these methods have the disadvantages resulting from the use of a pathogenic micro-organsim such as E.coli.

In fact, as well as producing undesirable substances such as endotoxins, this bacterium can express human beta interleukin-1 intracellularly as an insoluble protein.

As is known, this is due to the fact that heterologous protein expressed with high yields in a foreign environment assumes an inappropriate twist or configuration which is manifested in the formation of inclusions within the bacteria. The recovery and purification of beta hIL-1 therefore requires the host cells to be broken and the protein required to be separated from the cell material and from the contaminant proteins which may be present in considerable percentages in the inclusions.

Typically, the included bodies can be made soluble only with strong denaturing agents such as urea and guanidinium hydrochloride.

Purification methods which use these denaturing agents are characterised by a combination of two or more chromatographic techniques and produce a recombinant beta interleukin-1 with a good degree of purity. However, for reasons which are not completely clear, but probably due to the atypical rewinding of the protein, the levels of specific activity of the purified beta hIL-1 do not exceed values of approximately 6 x 10⁶ Units/mg.

Patent application No. GB 2186580 describes a method for the purification of the soluble fraction of recombinant human alpha interleukin-1 produced from engineered E.coli cells by a combination of gel filtration on Sephacryl S-200 and chromatography on DEAE-cellulose.

However, with this method, an alpha IL-1 is obtained which has a specific activity of 0.4 to 1 x 10⁷ U/mg of protein with a yield, measured as the biological activity, of 50%.

However, this method does not enable the complete removal of the endotoxins whose presence is undesirable in a product for use in human therapy.

This method, which uses transformed E. coli cells, is also limited by the fact that only some of the alpha IL-1 is expressed in a soluble form.

S.T. Motley and S. Graham (Genetics and Biotechnology of Bacilli, Vol. 2, pp. 371-375, 1987) disclose a process for the expression and secretion in Bacillus subtilis of a human beta IL-1 having a specific activity value comparable to those reported above.

The object of the present invention is therefore to develop a simple and economically advantageous method for the preparation of homogeneous human beta interluekin-1 with a high yield, without endotoxins, and with a better specific activity than the prior art.

According to the present invention, this object is achieved by the provision of a hitherto unknown method which uses an engineered micro-organism selected from the Bacillus group.

In fact, as well as not producing endotoxins, these bacteria can express human beta interleukin-1 in a completely soluble form, thus enabling its purification to homogeneity by means of a single chromatographic step and the production of a protein with a specific activity of at least 1 x 10⁸ units/mg of protein and with a yield of approximately 70%.

A subject of the present invention is therefore constituted by a method for the preparation of recombinant human beta interluekin-1 as a homogeneous protein without endotoxins and with a specific activity of at least 10⁸ Units/mg, which includes the purification, in a single chromatographic step, of the soluble fraction containing the protein produced by the breakdown of engineered Bacillus cells grown under suitable conditions.

Another subject of the present invention is represented by the above method, further comprising the step of formulating a pharmaceutical composition containing a therapeutically effective quantity of the purified recombinant human beta IL-1 useful in the treatment and prevention of diseases.

Another subject of the present invention is constituted by a recombinant DNA molecule obtained by the joining of a replicable cloning vector with expression in Bacillus to the gene which codes for human beta interleukin-1, and a micro-organism selected from the Bacillus group transformed by the recombinant DNA molecule.

Further objects of the present invention will become clear from a reading of the following description and examples.

In particular, the method according to the present invention comprises:
a) the cultivation in a suitable culture medium of a host micro-organism selected from the Bacillus genus, transformed by a recombinant DNA molecule containing the gene which codes for human beta interleukin-1,
b) the break-down of the cells and the separation of the soluble fraction from the insoluble cell fraction by centrifuging and finally
c) the purification to homogeneity of the human beta interleukin-1 from the soluble fraction in a single step by means of ion-exchange chromatography with the use of 20mM Tris-HCl, pH 7.5 buffer as the eluent.

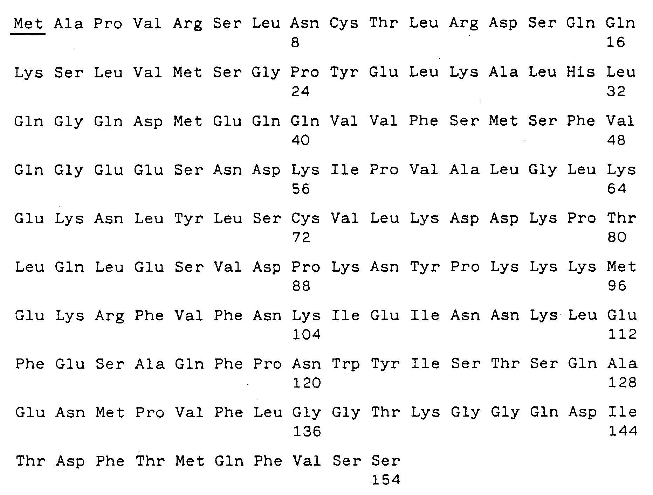

The recombinant human beta interleukin-1 produced by the method of the present invention is characterised by the aminoacid sequence shown in Figure 1.

According to the method of the present invention, strains of Bacillus subtilis, Bacillus amyloliquefaciens, Bacillus cereus, Bacillus brevis or Bacillus stearothermophilus, deposited at a collection centre such as, for example, the American Type Culture Center and available to the public, may be used as host micro-organisms in stage a).

The SMS 118 strain of Bacillus subtilis (leu, pyr, D1, npr⁻ , spr⁻ ) transformed by a recombinant DNA molecule obtained by the joining of a Bacillus cloning vector to the gene which codes for beta hIL-1 was preferably used for the purposes of the present invention. Cloning vectors suitable for the purposes of the present invention may be selected from the Bacillus plasmids generally used in the art.

The plasmid pSM 214 ATCC 67320, which is characterised by good stability in B. subtilis and can induce efficient expression of the heterologous protein, was preferably used. This plasmid contained the functional replication origins of puB110 and pBR322 which enable the replication, in B.subtilis and in E.coli, of the Km, Bla and Cat genes which code respectively for resistance to kanamycin, ampicillin and chloramphenicol, a strong synthetic promotor which directs the transcription of a dicystronic messenger RNA (mRNA) including the sequences of the Bla and Cat genes and, finally, the tₒ terminator of the lambda phage of E.coli, situated downstream of the Cat gene.

The removal of the Bla gene from this plasmid with EcoRI and HindIII restriction enzymes, and the subsequent insertion of a heterologous gene in the sites, enables the construction of a recombinant DNA molecule in which transcription is ensured by the presence of the single promotor, with selection on chloramphenicol.

According to the present invention, the heterologous gene is that which codes for beta hIL-1.

This gene may be obtained as cDNA from the total RNA extracted from the producer cells of beta hIL-1, as described by Auron et al ((1984) Poc. Natl. Acad. Sci. USA 81, 7907-7911) and by March et al ((1984) Nature 15, 641-646).

In particular, a 600 bp (base pair) fragment including the beta hIL-1 gene without the 5′ terminal sequence which codes for the first 11 aminoacids of the protein was isolated from the plasmid pUC8 - beta hIL-1 which carries the 604 bp Sau3A fragment which codes for beta IL-1 described by Mach et al, by digestion with HpaII and PstI restriction enzymes.

A 36 bp oligonucleotide which codes for the 11 aminoacids which the cutting with HpaII had removed from the DNA was then synthesised.

The ATG triplet which codes for methionine (Met), which is essential for starting the translation of all proteins, and the sequence generated by the cutting with EcoRI, were then positioned on the 5′ terminal part of the oligonucleotide.

According to the present invention, the oligonucleotide was ligated to the 600 bp DNA fragment in ligation mixture in the presence of T4 DNA ligase.

The resulting DNA fragment, constituted by 640 bp was separated by gel electrophoresis and then inserted in the pSM214 vector digested with EcoRI and PstI restriction enzymes.

The reaction was carried out in ligation mixture in the presence of T4 DNA ligase at 14°C for one night. The ligation mixture was then used for transforming SMS118 B. subtilis cells made competent by Contente and Dubnau's method (Mol. Gen. Genet. 167, 258 (1979)).

The cells thus transformed were selected on a suitable culture medium made selective by the addition of chloramphenicol.

The recombinant plasmid was isolated from one of the positive clones and, upon electrophoretic analysis and sequencing, showed the expected characteristics.

This plasmid, designated by the refrence pSM 261, was deposited as B.subtilis SMS118 (pSM 261) at the American Type Culture Center, and the number ATCC 67743 was assigned to it on 19. 7.1988.

According to the present invention, the B. subtilis SMS118 cells transformed by pSM216 were cultivated in a suitable medium, such as veal infusion broth (Difco) for example, in the presence of chloramphenicol at the temperature and for the period of time necessary to achieve the expression of the gene inserted in the cell.

The total proteins were then extracted from the broken Bacillus subtilis SMS 118 (pSM261) and the human beta interleukin-1 was separated and purified.

Various methods may be used for breaking the cells, such as, for example chemical treatment, enzymatic degradation with lysozyme, physical treatment with glass balls or the like, or preferably, sonication.

In practice, according to the method of the present invention, after separation from the culture medium by centrifuging, the cells were washed repeatedly in a suitable buffer and then broken by means of 2 or more consecutive passes through a French Press at 18,000 p.s.i.

The homogenate thus obtained was then centrifuged at 38,000 revolutions for 30 minutes in order to separate the soluble fraction from the insoluble cell material, which according to the prior art methods, contained the greater part of the beta hIL-1 expressed by the transformed E.coli cells.

The supernatant liquid was then analysed to measure the total proteins by the BIO-RAD micro-method, producing a value of approximately 6.2 mg/ml.

Moreover, both the soluble fraction and the insoluble fraction were examined, after electropheoesis on 12.5% polyacrylamide gel, both by dyeing with Coomassie Blue ("Gel Electrophoresis of Proteins: A Practical Approach" edited by B.D. Hames and D. Rickwood, IRL Press Limited) and by means of immunoblotting after transfer onto nitrocellulose.

The results obtained showed the presence, only in the soluble fraction, of a protein having a molecular weight of approximately 17,000 daltons and capable of reacting specifically with anti-beta IL-1 antibodies.

According to the present invention, the recombinant beta hIL-1 was then purified from the soluble fraction thus obtained by ion-exchange chromatography.

A DEAE-cellulose column, equilibrated with 20mM Tris-HCl, pH 7.5 buffer, was preferably used.

The proteins were then eluted from the column with the same buffer as was used for its equilibration at a flow rate of 25 ml/hour, the absorbance of the eluate being monitored continuously at 280 nm.

The fractions containing the eluted proteins were collected and examined by gel electrophoresis on sodium dodecylsulphate-polyacrylamide (SDS-PAGE) as described by Laemmli (Nature, 277, 680-685 (1970)).

Those containing beta hIL-1, which showed a single narrow band, were combined and concentrated by filtering on a membrane, producing a solution with a proteinaceous content of 2.5 mg/ml.

The purity of the beta hIL-1 was confirmed by the presence of a single narrow band on SDS-PAGE and a single peak on HPLC.

The biological activity of the beta IL-1 purified according to the method of the present invention was measured by the test described by Kaye et al. (J. Immunolog. (1984) 13, 1339-1345) with the use of a standard beta IL-1 with a specific activity of 1 x 10⁷ U/mg of protein.

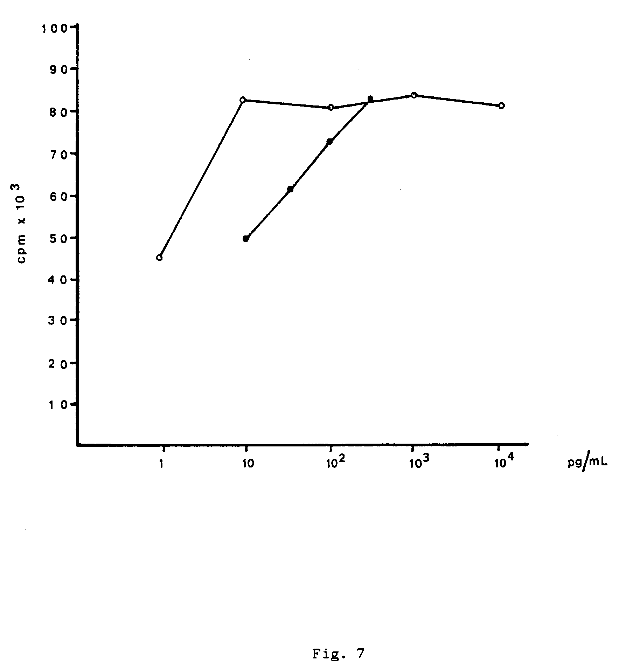

As shown in Figure 7, the beta IL-1 showed a biological activity 5-10 times greater than that of the standard.

The molecular weight of the homogeneous beta hIL-1 determined by electrophoresis on polyacrylamide gel was approximately 17,000 daltons.

The specific activity of the purified product was 1 x 10⁸ Units/mg of protein.

The homogeneous human beta interleukin-1 obtained by the method of the present invention is therefore characterised in that it was isolated in the form of a single peak and produced a single narrow band on electrophoresis on SDS-polyacrylamide gel in the presence of 2-mercaptoethanol.

The homogeneous beta hIL-1 thus obtained can be used as a drug for stimulating the immune system of a patient, for example to improve the defences of a host against pathogens, and as an adjuvent in a vaccine.

Other clinical uses include the treatment of auto-immune diseases and the activation of repair mechanisms in cases of tissue damage.

The human beta interleukin-1 thus obtained can be administered to man for the clinical uses indicated above.

The administration may be carried out by conventional methods, such as intramuscular, intravenous and subcutaneous introduction.

Naturally the dosage required may vary according to the condition to be treated, the gravity of the case, the duration of the treatment and the methods of administration.

A pharmaceutical dosage form can be produced by the reconstitution of lyophilised beta hIL-1 in a sterile solution before use.

Moreover, as is well known to an expert in the art, stabilising buffers and bacteriostatic substances and other conventional additives may be used in the pharmaceutical compositions.

The following experimental examples are illustrative of the invention and are not limiting.

### BRIEF DESCRIPTION OF THE DRAWINGS

- Fig. 1: The sequence of beta IL-1 deduced from the nucleotide sequence of the gene cloned and expressed in B. subtilis. Methionine is underlined and is the only aminoacid which does not form part of the sequence of natural beta IL-1.

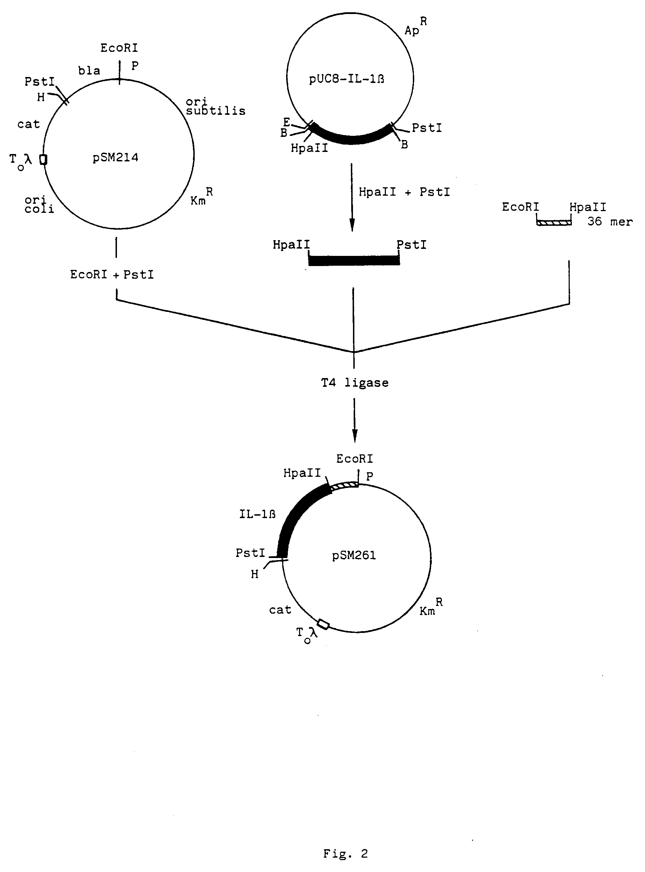

- Fig.2: A diagram of the strategy for cloning the beta IL-1 gene in B. subtilis.
E - EcoRI
B = BamHI
H = HindIII
P = Promoter
- Fig.3A:: 12.5% polyacrylamide gel, in which the total proteins extracted from B. subtilis cells had been isolated, dyed with Coomassie Blue.
1: SMS118; 2: SMS118 (pSM 214); 3: molecular weights standard; 4: SMS 118 (pSM 261).
- B:: An immunoblot of the total proteins extracted from B. subtilis cells and separated on 12.5% polyacrylamide gel.
1: SMS118; 2: SMS118 (pSM 214); 3: SMS118 (pSM 261). The traces indicate the position of beta IL-1.

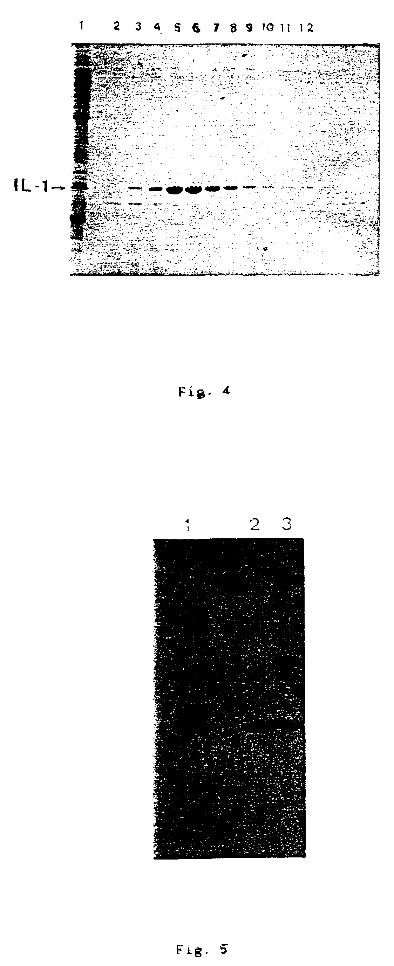

- Fig.4: Polyacrylamide gel of the total proteins present in the crude extract of B. subtilis (1) and in the fractons obtained after ion-exchange chromatography on DEAE-Cellulose. (2): fraction 8; (3): fraction 10; (4): fraction 12: (5): fraction 14; (6) fraction 16; (7): fraction 18; (8): fraction 20; (9): fraction 22; (10): fraction 24; (11) fraction 26; (12): position 28.
- Fig.5: Electrophoresis of the purified beta IL-1 sample in the presence (column 2) and in the absence (Column 1) of beta-mercaptoethanol. (Column 3): standard beta IL-1 (SCLAVO).

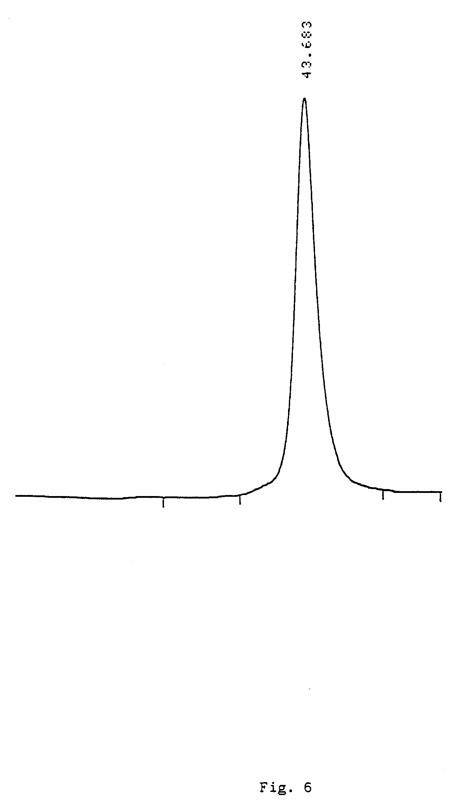

- Fig.6: A chromatogram of beta IL-1 purified on DEAE-cellulose, obtained by HPLC (reverse-phase). For technical details see example 2.
- Fig.7: The incorporation of marked thymidine by D10 cells after the addition of purified beta IL-1 (- 0 -) and standard beta IL-1 (- 0 -).

### Example 1

### Expression of beta interleukin-1 in Bacillus subtilis

### a) Construction of the recombinant plasmid pSM261

1 µg of the plasmid pSM 214 was digested in 20 µl of a solution containing 50 mM Tris-HCl pH 7.5, 10 mM MgCl₂, 50 mM NaCl and 1 unit of EcoRI and PstI (Boehringer) at 37°C for 1 hour. The enzymes were then made inactive at 65°C for 10 minutes.

10 µg of the plasmid pUC8 - beta IL-1 were digested in 100 µl of the solution described above with 10 units of HpaII and PstI at 37°C for 1 hour.

The enzymes were made inactive at 65°C for 10 minutes.

The 600 base pair (bp) HpaII-PstI fragment carrying the gene which codes for beta IL-1 was then separated from the digestion mixture by electrophoresis on polyacrylamide gel and subsequent elution (Maxam and Gilbert (1980) Methods in Enzymology 65, 499-560).
with the use of a Beckman DNA Synthesizer System One plus, a 36 bp oligonucleotide was then synthesized which codes for the first 11 aminoacids of beta IL-1, which the cutting with HpaII had removed from the DNA.

The ATG methionine triplet and the sequence generated by the cutting with the EcoRI restriction enzyme were also positioned on the 5′ terminal part of the oligonucleotide whose sequence is: 
5' AATTCTTATGGCACCTGTACGATCACTGAACTGCACGCTC 3'
3' GAATACCGTGGACATGCTAGTGACTTGACGTGCGAGGC 5'
10 20 30 6
0.5 µg of the synthetic oligonucleotide were then ligated with 200 µg of the 600 bp HpaII - PstI fragment in 50 µl of a solution containing 66 mM Tris-HCl pH 7.5, 10 mM MgCl₂, 10 mM dithiothreitol (DTT), 1 mM ATP and 0.1 unit of T4 DNA ligase.

The reaction was carried out at 14°C for 14 hours and the enzyme was then made inactive at 65°C for 10 minutes.

The reaction mixture was then loaded onto polyacrylamide gel and the 640 bp EcoRI-PstI DNA fragment was separated and eluted as described above.

After elution, 100 ng of the fragment were resuspended in 20 ul of TE (10mM Tris-HCl pH 7.5, 1 mM EDTA).

2µl of the solution containing 100 ng of pSM 214 digested with EcoRI and PstI were then added to 50 µl of a solution of 66 mM Tris-HCl pH 7.5, 10 mM MgCl₂, 10 10 mM DTT, 1 mM ATP, containing 50 ng of the EcoRI-PstI DNA fragment and ligated in the presence of 0.1 unit of T4 DNA ligase at 14°C for 18 hours.

After the enzyme had been made inactive, the entire ligation mixture was used for transforming B. subtilis SMS 118 (leu, pyrDI, npr⁻, spr⁻) cells made competent by the method described by Contente and Dubnau (Mol. Gen. Genet., 167, 251-258 (1979).

The transformants were selected on VY (DIFCO Veal Infusion Broth) medium containing 5 µg/ml of chloramphenicol at 37°C for 18 hours.

The transformant clones carrying the expected hybrid plasmid were identified by the rapid extraction of the plasmid DNA as described by Rodriguez and Tait ("Recombinant DNA Techniques: An Introduction", pp. 50-51, Addison-Wesley Publishing Company).

The plasmid DNA of the various clones was digested with the EcoRI and PstI enzymes under the conditions recommended by the supplying company (Boehringer) and analysed by means of 0.8% agarose gel (Maniatis et al (1982) Molecular Cloning: A Laboratory Manual, Cold Spring Harbor).

One of the plasmids which, after cutting with the enzymes gave rise to two fragments of 640 bp and 7000 bp respectively, was designated pSM 261 and studied further.

In order to confirm the correct sequence of the 5′ terminal region of the beta IL-1 gene, after treatment with alkaline phosphatase, the 640 bp fragment was marked at the exposed 5′ end of the EcoRI site and sequenced (Maxam and Gilbert (1980) Methods in Enzymology).

Analysis of the sequence confirmed the expected gene structure.

### b) Verification of the expression of beta IL-1 in B. subtilis SMS118 (pSM261).

The total proteins were extracted from 1 ml of a cell culture of SMS118 (pSM261) grown at 37°C for 16 hours in VY medium containing 5 µg/ml of chloramphenicol by the following method. The cells were centrifuged for 1 minute in an Eppendorf centrifuge, washed with an equal volume of 30 mM Tris-HCl pH 7.6, 50 mM NaCl and resuspended in 100 µl of SET buffer (20% sucrose, 30 mM Tris-HCl pH 7.6, 1 mM EDTA) containing 1mg/ml of lysozyme.

The cell lysis was carried out at 37°C for 15 minutes. 150 µl of a buffer containing 125 mM Tris-HCl pH 6.8, 3% SDS, 3% beta mercaptoethanol and 20% glycerol were then added and the mixture was incubated at 100°C for 5 minutes. 20 µl portions were loaded onto 15% polyacrylamide gel after the addition of 2 µl of dye (0.25% bromophenol blue, 40% sucrose). After electrophoresis had been carried out at 20 mA for 3 hours, the proteinaceous bands were displayed by dyeing with Coomassie blue and, in parallel, transferred onto nitrocellulose filters (Schleicher and Schull 45 µm) and treated with rabbit anti-beta IL-1 antibodies and goat anti-rabbit-antibody antibodies complexed with peroxydase (Miles).

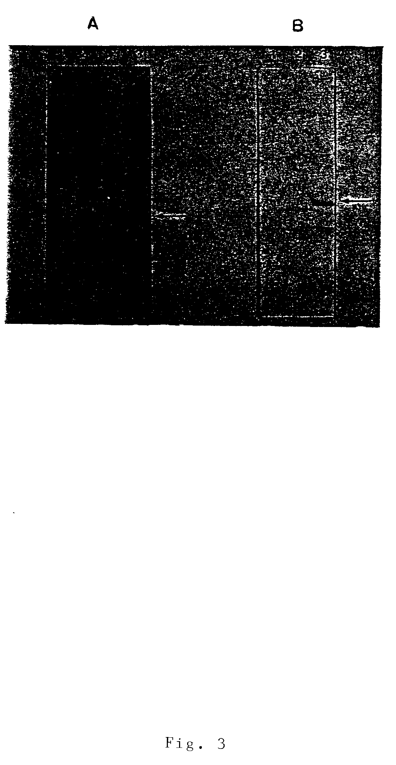

After the dyeing with Coomassie blue, there was the clear presence of a protein with a molecular weight of 17,000, which was absent from the extracts of B.subtilis SMS118 and SMS118 (pSM214) (Fig. 3A). This protein reacts specifically with the anti-beta IL-11 antibodies (Fig. 3B). Moreover, densitometric analysis carried out on the same gel dyed with Coomassie blue indicates that the quantity of beta IL-1 produced by B. subtilis SMS118 (pSM261) corresponds to 6% of the total proteins.

It was also shown that the insoluble cell fraction obtained by the centrifuging of the cell lysate at 6500 rpm for 10 minutes did not contain beta IL-1: the protein was therefore expressed in a completely soluble form.

### Example 2.

### Purification of recombinant human beta interleukin-1

4 g of wet cells were collected by centrifuging from 1 litre of B. subtilis SMS118 (pSM261) culture.

These cells were washed and broken down as described in Example 1.

The homogenate thus obtained was centrifuged in a Sorvall centrifuge at 38,000 rpm for 30 minutes and the supernatant liquid (29 ml) recovered.

The measurement of the total proteins, carried out by the BIO-RAD micro-method lead to the determination of 6.2 mg/ml of proteins.

20 ml of the supernatant liquid were then loaded into a 1.6 x 35cm DEAE-cellulose (DE-52 Whatman) column previously equilibrated with 20 mM Tris-HCl pH 7.5 buffer.

The proteins were eluted from the column with the use of the same buffer as described above at a flow rate of 25 ml/hour.

The elution was carried out with continuous monitoring of the absorbance of the eluate at 280 nm.

The fractions containing the eluted proteins were collected and examined electrophoretically on SDS-PAGE (Laemmli, (1970) Nature, 227 68-685) (Figure 4).

Those containing beta hIL-1 (fractions 14-19), which showed a single narrow band, were recombined producing 65 ml of a solution with a proteinaceous content of approximately 80 µg/ml.

The solution was then concentrated by filtering on YM-10 membrane (Amicon) producing 2 ml of solution containing 2.5 mg/ml of protein (yield 70%).

The purity of the beta hIL-1 thus obtained was confirmed by electrophoresis on 12.5% SDS-polyacrylamide gel in the presence (Figure 5, column 2) and in the absence (Figure 5, column 1) of the beta-mercaptoethanol reducer.

After the gel had been dyed with Coomassie Blue, the sample appeared to be composed of a single protein type.

In order further to confirm the purity of the beta IL-1 sample, 5 µl of the protein solution were loaded into a 4.6 x 250 mm ALTEX ULTRASIL-ODS type colum (cat. No. 256-25, Beckman) and subjected to chromatography with the use of a Beckman-HPLC chromatograph, Model 110B by means of a gradient of from 0.1% TFA (trifluoracetic acid) in H₂0 to 0.1% TFA in 80% CH₃CN (acetonitrile) (F.E. Dewhirst et al., (1985) J. Immunol. 135, 2562-2568).

As shown in Figure 6, the beta IL-1 sample was eluted in a single peak with a retention time of 43.683 minutes.

The biological activity of the beta IL-1 sample was measured by the test described by Kaye et al. (J. Immunology (1984) 133, 1339-1345). In particular, D10 cells which had just been thawed were distributed (2X10⁴/dish) in flat-bottomed Cluster⁹⁶ plates micro-dishes (Costar, Cambridge Massachusetts) in RPMI-1640 culture medium (GIBCO, Grand Island, NY) containing 25 mM HEPES, 50 µg/ml gentamicin, 2 mM L-glutamin, 10% uncomplemented bovine foetal serum (HyClone, Sterile Systems, Logan, Utah) and 6 x 10⁻⁵ M 2-mercaptoethanol.

Graduated amounts of standard IL-1 or of the sample, diluted with the same medium, were added to the dishes in triplicate. ConA (concanovaline A) was also added to a final concentration of 2.5 µg/ml. The final volume of each dish was 0.2 ml.

Incubation was carried out for 48 hours at 37°C (moist, 5% CO₂). In order to measure the multiplication, 0.5 µCi of thymidine tritiate (Amersham, specific activity 2 Ci/mmole) were added per dish and then these were incubated for another 16-18 hours.

After the cells had been collected from the dishes with a multiple cell harvester (Skatron, Lier, Norway), the radioactivity incorporated by the mutliplying cells was evaluated by liquid scintillation.

As shown in Figure 7, the maximum incorporation of marked thymidine by the D10 cells was achieved with the addition of 10 pg of the purified beta IL-1, a concentration 5-10 times less than that required for the standard beta IL-1 (SCLAVO) (having a specific activity of 10⁷ U/mg).

Table I shows the purification scheme used with the relative yields and purification factors.

**TABLE I**

| | mg protein/per gram of cells | activity (U/mg) | Estimated Yield⁽¹⁾ (%) | Purification Factor |
|---|---|---|---|---|
| Extract | 45 | 2 x 10⁶ | | |
| DEAE Column | 1.86 | 1 x 10⁸ | 70% | 20 |

| | | | | |
|---|---|---|---|---|
| (1) The yield was evaluated on the basis of the concentrations of the proteinaceous preparations measured by densitometric analysis of the polyacrylamide gel. | | | | |

## Claims

1. A method for the preparation of homogeneous recombinant human beta interleukin-1 without endotoxins and with a specific activity of at least 1-10⁸ units/mg of proteins, which comprises:
a) the cultivation in a suitable culture medium of a host micro-organism of the Bacillus genus transformed by a recombinant DNA molecule containing the gene which codes for human beta interleukin-1;
b) the separation of the soluble fraction from the insoluble cell fraction by centrifuging, after the break-down of the host cells;
c) the purification to homogeneity of the recombinant human beta interleukin-1 from the soluble fraction in a single step by means of ion-exchange chromatography.

2. A method according to Claim 1, in which the host micro-organism is a strain of Bacillus subtilis.

3. A method according to Claim 1, in which the recombinant DNA molecule is produced by the joining of a replicable cloning vector with expression in Bacillus to the gene which codes for human beta interleukin-1.

4. A method according to Claim 3, in which the cloning vector is a plasmid replicable in Bacillus subtilis.

5. A method according to Claim 4, in which the plasmid is pSM214 ATCC 67320.

6. A replicable recombinant DNA molecule (pSM 261) with expression in Bacillus subtilis, deposited as ATCC 67743.

7. A method according to Claim 1, in which the culture medium in step a) is veal infusion broth and the culture is carried out in the presence of chloramphenicol at a temperature of from 25° to 40°C.

8. A method according to Claim 1, in which the cells are broken in step b) by physical treatment in a French Press at 18,000 p.s.i.

9. A method according to claim 1, in which the ion-exchange chromatography in step c) is carried out with the use of a DEAE cellulose column with elution in 20 mM Tris-HCl pH 7.5 buffer.

10. A method according to claims 1-9 further comprising the step of formulating a pharmaceutical composition containing a therapeutically effective quantity of the purified recombinant human beta IL-1 useful in the treatment and prevention of diseases.

## Patentansprüche

1. Verfahren zur Herstellung von homogenem rekombinantem Human-β-Interleukin-1 ohne Endotoxine, das eine spezifische Aktivität von mindestens 1x10⁸ Einheiten/mg Proteine aufweist, das umfaßt:
a) die Kultivierung eines Wirts-Mikroorganismus des Genus Bacillus, der durch ein rekombinantes DNA-Molekül transformiert worden ist, welches das Gen enthält, das für Human-β-Interleukin-1 codiert, in einem geeigneten Kulturmedium;
b) die Abtrennung der löslichen Fraktion von der unlöslichen Zellfraktion durch Zentrifugieren nach dem Zerbrechen (Zerstören) der Wirtszellen; und
c) die Reinigung des rekombinanten Human-β-Interleukins-1 aus der löslichen Fraktion bis zur Homogenität in einer einzigen Stufe durch Ionenaustauschchromatographie.

2. Verfahren nach Anspruch 1, in dem der Wirts-Mikroorganismus ein Stamm von Bacillus subtilis ist.

3. Verfahren nach Anspruch 1, in dem das rekombinante DNA-Molekül hergestellt wird durch Vereinigen eines replizierbaren Klonierungsvektors mit Expression in Bacillus mit dem Gen, das für Human-β-Interleukin-1 codiert.

4. Verfahren nach Anspruch 3, in dem der Klonierungsvektor ein in Bacillus subtilis replizierbares Plasmid ist.

5. Verfahren nach Anspruch 4, in dem das Plasmid pSM214 ATCC 67320 ist.

6. Replizierbares rekombinantes DNA-Molekül (pSM 261) mit Expression in Bacillus subtilis, das hinterlegt ist unter der Nr. ATCC 67743.

7. Verfahren nach Anspruch 1, in dem das Kulturmedium in der Stufe (a) eine Kalbsinfusionsbrühe ist und die Kultivierung in Gegenwart von Chloramphenicol bei einer Temperatur von 25 bis 40°C durchgeführt wird.

8. Verfahren nach Anspruch 1, in dem die Zellen in der Stufe (b) durch physikalische Behandlung in einer French-Presse bei 18 000 psi zerbrochen (zerstört) werden.

9. Verfahren nach Anspruch 1, in dem die Ionenaustauschchromatographie in der Stufe (c) unter Verwendung einer DEAE-Cellulose-Säule unter Elution in einen 20 mM Tris-HCl pH 7,5-Puffer durchgeführt wird.

10. Verfahren nach den Ansprüchen 1 bis 9, das außerdem umfaßt
die Formulierung einer pharmazeutischen Zusammensetzung, die eine therapeutisch wirksame Menge des gereinigten rekombinanten Human-β-IL-1 enthält, die geeignet ist für die Behandlung und Prävention von Erkrankungen.

## Revendications

1. Procédé de préparation de bêta-interleukine-1 humaine recombinante homogène dépourvue d'endotoxines et ayant une activité spécifique d'au moins 1-10⁸ unités/mg de protéines, qui comprend :
a) la culture dans un milieu de culture convenable d'un microorganisme hôte appartenant au genre Bacillus, transformé avec une molécule d'ADN recombinant contenant le gêne qui code pour la bêta-interleukine-1 humaine ;
b) la séparation de la fraction soluble de la fraction cellulaire insoluble par centrifugation, après la dissociation des cellules-hôtes ;
c) la purification jusqu'à homogéneité de la bêta-interleukine-1 humaine recombinante à partir de la fraction soluble en une seule étape par chromatographie d'échange d'ions.

2. Procédé suivant la revendication 1, dans lequel le microorganisme hôte est une souche de Bacillus subtilis.

3. Procédé suivant la revendication 1, dans lequel la molécule d'ADN recombinant est produite par jonction d'un vecteur de clonage réplicable avec expression dans le genre Bacillus au gêne qui code pour la bêta-interleukine-1 humaine.

4. Procédé suivant la revendication 3, dans lequel le vecteur de clonage est un plasmide réplicable dans Bacillus subtilis.

5. Procédé suivant la revendication 4, dans lequel le plasmide est pSM214 ATCC 67320.

6. Molécule d'ADN recombinant réplicable (pSM 261) avec expression dans Bacillus subtilis, déposée sous la référence ATCC 67743.

7. Procédé suivant la revendication 1, dans lequel le milieu de culture dans l'étape a) est un bouillon d'infusion de viande de veau et la culture est effectuée en présence de chloramphénicol à une température de 25° à 40°C.

8. Procédé suivant la revendication 1, dans lequel les cellules sont dissociées dans l'étape b) par traitement physique dans une presse French sous une pression de 18 000 psi.

9. Procédé suivant la revendication 1, dans lequel la chromatographie d'échange d'ions dans l'étape c) est effectuée au moyen d'une colonne de DEAE-cellulose avec élution dans un tampon Tris-HCl 20 mM à pH 7,5.

10. Procédé suivant les revendications 1 à 9, comprenant en outre l'étape de formulation d'une composition pharmaceutique contenant une quantité thérapeutiquement efficace de la bêta-IL-1 humaine recombinante purifiée utile dans le traitement et la prévention de maladies.
